# EUROPEAN PATENT SPECIFICATION

(11) **EP 1 105 161 B1**
(45) Date of publication and mention of the grant of the patent: **02.04.2008**
(21) Application number: 99941024.4
(22) Date of filing: 10.08.1999
(51) Int. Cl.: A61K 47/48

(54) **PHOSPHORYLATED POLYMERS AND CONJUGATES THEREOF**
PHOSPHORYLIERTE POLYMERE UND DEREN KONJUGATE
POLYMERES PHOSPHORILES ET LEUR CONJUGUES

(30) Priority: 10.08.1998 US 131472; 10.08.1998 US 95875 P
(43) Date of publication of application: 13.06.2001
(73) Proprietor: Poly-Med Inc., Anderson, SC 29625 (US)
(72) Inventor: SHALABY, Shalaby, Wahba, Pendleton, SC 29670 (US); CORBETT, Joel, Thomas, Seneca, SC 29672 (US)
(74) Representative: Taylor, Kate Laura
(86) International application number: PCT/US1999/018146
(87) International publication number: WO 2000/009166

(56) References cited:
- EP-A- 0 326 148
- EP-A- 0 375 659
- EP-A- 0 407 617
- EP-A- 0 449 249
- EP-A- 0 722 966
- WO-A-96/00760
- WO-A-96/31220
- WO-A-97/19948
- DE-C- 4 435 082
- GB-A- 1 350 225
- GB-A- 2 145 422
- US-A- 4 041 223
- US-A- 4 764 604
- US-A- 5 079 337
- US-A- 5 183 809
- US-A- 5 310 879
- US-A- 5 525 326
- US-A- 5 536 445
- US-A- 5 635 216
- US-A- 5 654 422
- US-A- 5 672 659
- US-A- 5 725 881
- R.L.SHOGREN: "Complexes of starch with telechelic poly(e-caprolactone) phosphate" CARBOHYDRATE POLYMERS, vol. 22, 1993, pages 93-98, XP000881303
- DATABASE WPI Derwent Publications Ltd., London, GB; AN 1984-210427[25] XP002140521 & JP 59 122416 A (LION CORP), 14 July 1984 (1984-07-14)
- DATABASE WPI Derwent Publications Ltd., London, GB; AN 1997-063618[06] XP002140522 & KR 9 502 607 B (SAMYANG CO LTD)
- PATENT ABSTRACTS OF JAPAN vol. 13, no. 95, 6 March 1989 (1989-03-06) & JP 63 273679 A (BIO MATERIAL YUNIBAASU:KK), 10 November 1988 (1988-11-10)
- TSENG YIN CHAO ET AL.: "Effect of poly (D,L-Lactide) addition to 2-cyanoacrylates in their physical properties and toxicity" J. BIOACT. COMPAT. POLYM., vol. 4, no. 2, 1989, pages 101-109, XP000915928
- TSENG YIN CHAO: "Medical application of cyanoacrylates as surgical adhesives." JINKO ZOKI , vol. 18, no. 1, 1989, pages 409-413, XP000915929

## Description

### Background of the Invention

The present invention is directed to absorbable polyesters comprising one or more monophosphate functionality; a conjugate comprising the foregoing polyester and a peptide and/or a bioactive agent; microparticles comprising an absorbable polyester; a conjugate comprising the microparticles and a peptide and/or a bioactive agent; an acylated or alkylated polysaccharide having one or more monophosphate functionality; a conjugate comprising the acylated or alkylated polysaccharide and a peptide and/or a bioactive agent; and pharmaceutical compositions thereof.

Phosphorous-containing heterochain polymers have been cited in the patent and technical literature in conjunction with (1) flame-retardant and/or hydrophilic polyamides (Shalaby, S.W., et al., J. Polym. Sci., Polym. Chem. Ed., 12, 2917 (1974); Shalaby, S.W., et al., J. Polym. Sci., Polym. Chem. Ed., 13, 669 (1975); Shalaby, S.W., et al., J. Polym. Sci., Polym. Chem. Ed., 14, 2675 (1976); Shalaby, S.W. and McCaig, S., U.S. Patent No. 5,491,198; Shalaby, S.W. and Rogers, K.R., U.S. Patent No. 5,558,517); (2) flame-retardant polyesters (Heffner, R.E., U.S. Patent No. 4,420,587; Koch, P.J., et al., J. Appl. Polym. Sci., 19, 227 (1975); Japanese Pat. 51-40432); (3) flame-retardant polyurethanes and epoxy resins (German Patent DE 1,292,862, US Pat. 3,321,555); (4) thermally stable polyesters with chain end-groups reacted with phosphonyl thiocyanate (U.S. Patent No. 3,838,045); (5) phosphorylated cellulose as a cation-exchanger (DD 286600); and (6) phosphorylated polyesters as semiconducting materials (SU, 672878). However, there is no disclosure in the art of incorporation of phosphate groups at available hydroxy endgroup sites of absorbable polyesters, which sites are typically present at one or two terminals of the chain. This is not surprising since conditions of chemical reactions known for hydroxy group phosphorylation could cause hydrolysis of the highly reactive absorbable polyester chains. Meanwhile, there has been great interest in developing carboxyl-bearing absorbable polyesters for use in forming conjugates with bioactive polypeptides as controlled release systems therefor (Shalaby, S.W., et al., U.S. Patent No. 5,672,659).

Therefore, there is an incentive to make directly phosphorylated absorbable polyesters without significantly causing chain degradation to obtain novel controlled release systems.

WO9711994.8 describes a dispersant which is a phosphate ester of a block copolymer of formula MeO(C₂H₄O)ₘ(PES)ₙ-H wherein PES is derived from ε-caprolactone.

US 5536445 describes a surfactant comprising a carboxylic acid ester or amide carrying a terminal strong acid group selected from carboxymethyl, sulphate, sulphonate, phosphate and phosphonate.

US 5672659 describes a composition including a polyester containing one or more free COOH groups ionically conjugated with a bioactive polypeptide comprising at least one effective ionogenic amine.

US 5079337 describes a water-soluble macro molecular conjugate of haemoglobin with a water-soluble polymer containing polar groups.

Shogren, Carbohydrate Polymers, 22: 93-98, (1993) describes complexes of starch with telechelic poly(ε-caprolactone) phosphate.

### Summary of the Invention

In one aspect, the present invention is directed to an absorbable polyester with at least one monophosphate functionality per absorbable polyester chain. The polymer chain is covalently linked to phosphoric acid by a single phosphate bond leaving two additional acidic groups available for conjugation or neutralization and the polyester chain comprises one or more monomers selected from the group consisting of L-lactie acid, D-lactic acid, DL-lactic acid, malic acid, citric acid, tartaric acid, , alkylene oxalate, cycloalkylene oxalate, alkylene succinate, β-hydroxybutyrate, glycolide, glycolic acid, L-lactide, D-lactide, DL.-tactide, meso-lactide, trimethylene carbonate, p-dioxanone, 1,5-dioxepar7-2-one and 1,4-dioxepan-2-one and any optically active isomers, racemates, or copolymers thereof. A more preferred embodiment of the foregoing invention is where the absorbable polyester further comprises one or more polyethylene glycol segments covalently linked to the polyester. A further aspect of the foregoing invention is directed to a. conjugate comprising the immediately foregoing absorbable polyester and a peptide and/or a bioactive agent, where the peptide and bioactive agent have at least one interactive amino group, wherein the monophosphate functionality forms a linkage with the amino group. A preferred embodiment of the foregoing conjugate is where the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-CysrThr NH₂ where the two Cys are bonded by a disulfide bond, or a pharmaceutically acceptable salt thereof.

A further aspect of the foregoing invention, this invention is directed to a conjugate comprising an absorbable polyester with at least one monophosphate functionality per absorbable polyester chain, wherein the polymer chain is covalently linked to phosphoric acid by a single phosphate bond leaving two additional acidic groups available for conjugation or neutralization and wherein the polyester chain comprises one or more monomers selected from the group consisting of L-lactic acid, D-lactic acid, DL-lactic acid, malic acid, citric acid, tartaric acid, ε-hydroxy caproic acid, ε-caprolactone, alkylene oxalate, cycloalkylene oxalate, alkylene succinate, β-hydroxybutyrate, glycolide, glycolic acid, L-lactide, D-lactide, DL-lactide, meso-lactide, trimethylene carbonate, p-dioxanone, 1,5-dioxepan-2-one and 1,4-dioxepan-2-one and any optically active isomers, racemates, or copolymers thereof and a peptide and/or a bioactive agent, where the peptide and bioactive agent have at least one interactive amino group, wherein the monophosphate functionality forms a linkage with the amino group. A preferred embodiment of the foregoing conjugate is where the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-Cys-Thr-NH₂ where the two Cys are bonded by a disulfide bond, or a pharmaceutically acceptable salt thereof.

In another aspect, the present invention is directed to a solid absorbable microparticle which comprises the foregoing absorbable polyester and having a surface, wherein more than one percent of the monophosphate functionality resides on the surface of the absorbable microparticle.

In another aspect, the present invention is directed to a conjugate comprising the foregoing absorbable microparticle, where the peptide and bioactive agent have at least one interactive amino group, wherein the monophosphate functionality forms a linkage with the amino group. A preferred conjugate is wherein the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-cys-Thr-NH₂ where the two Cys are bonded by a disulfide bond, or a pharmaceutically acceptable salt thereof.

In another aspect, the present invention is directed to a conjugate comprising a solid absorbable microparticle which comprises an absorbable polyester with at least one monophosphate functionality per absorbable polyester chain, wherein the polymer chain is covalently linked to phosphoric acid by a single phosphate bond leaving two additional acidic groups available for conjugation or neutralization and wherein the polyester chain comprises one or more monomers selected from the group consisting of L-lactic acid, D-lactic acid, DL-lactic acid, malic acid, citric acid, tartaric acid, ε-hydroxy caproic acid, ε-caprolactone, alkylene oxalate, cycloalkylene oxalate, alkylene succinate, β-hydroxybutyrate, glycolide, glycolic acid, L-lactide, D-lactide, DL-lactide, meso-lactide, trimethylene carbonate, p-dioxanone, 1,5-dioxepan-2-one and 1,4-dioxepan-2-one and any optically active isomers, racemates, or copolymers thereof and having a surface, wherein more than one percent of the monophosphate functionality resides on the surface of the absorbable microparticle and a peptide and/or a bioactive agent, where the peptide and bioactive agent have at least one interactive amino group, wherein the monophosphate functionality on the surface of the absorbable microparticle forms a linkage with the amino group. A preferred embodiment of the immediately foregoing conjugate is the conjugate wherein the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsutfonyl-Phe-Cys-Tyr-D-Trp-Lys-Abu-Cys-Thr-NH₂ where the two Cys are bonded by a disulfide bond, or a pharmaceutically acceptable salt thereof.

In another aspect, the present invention is directed to a pharmaceutical composition, comprising the foregoing conjugate and a pharmaceutically acceptable carrier.

The term "grafted" refers to a polyester graft originating from a heterocyclic monomer, such as lactide, glycolide, trimethylene carbonate and/or ε-caprolactone.

The term "absorbable" means that a water insoluble material such as a polymer which undergoes chain dissociation in the biological environment to water soluble by-products as a function of time and leaves hardly any residue at the site of implant or administration.

The instant application denotes amino acids using the standard three letter abbreviations known in the art, for example Ala = alanine.

The term "microparticle" as used herein, refers to the particles of absorbable polyester, which are preferably in essentially spherical form.

The term "monophosphate functionality" means that the polymer chain is covalently linked to phosphoric acid by a single phosphate bond leaving two additional acidic -OH groups available for conjugation or neutralization as depicted in the following structure: polyester-CHR-O-P(O)(OH)₂, where R is, for example H or CH₃.

The term "peptide" is meant to include peptides, polypeptides and proteins. Examples of peptides include but are not limited to growth hormone releasing peptide (GHRP), leutenizing hormone-releasing hormone (LHRH), somatostatin, bombesin, gastrin releasing peptide (GRP), calcitonin, bradykinin, galanin, melanocyte stimulating hormone (MSH), growth hormone releasing factor (GRF), amylin, tachykinins, secretin, parathyroid hormone (PTH), encephalon, endothelin, calcitonin gene releasing peptide (CRP), neuromedins, parathyroid hormone related protein (PTHrP), glucagon, neurotensin, adrenocorticothrophic hormone (ACTH), peptide YY (PYY), glucagon releasing peptide (GLP), vasoactive intestinal peptide (VIP), pituitary adenylate cyclase activating peptide (PACAP), motilin, substance P, neuropeptide Y (NPY), TSH, growth hormone, erythropoietin, granulocyte-colony stimulating factor, granulocyte-macrophage-colony stimulating factor, interferons, the LHRH analog p-Glu-His-Trp-Ser-Tyr-D-Trp-Leu-Arg-Pro-Gly-NH₂ (Peptide A), the somatostatin analog H-β-D-Nal-Cys-Tyr-D-Trp-Lys-Val-Cys-Thr-NH₂ where the two Cys are bonded by a disulfide bond (Peptide B), N-hydroxyethylpiperazinyl-acetyl-D-Phe-Cys- Tyr-D- Trp-Lys-Abu-Cys- Thr-NH₂ where the two Cys are bonded by a disulfide bond, or N-hydroxyethylpiperazinyl-ethylsulfonyl-Phe-Cys-Tyr-D-Trp-Lys-Abu-Cys-Thr-NH₂ where the two Cys are bonded by a disulfide bond (Peptide C), and analogs and fragments thereof or a pharmaceutically acceptable salt thereof.

The term "bioactive agent" is meant to include any chemical compound, including peptides, that can be administered to a patient for therapeutic or prophylactic purposes and, thus, includes pharmaceuticals.

The term "interactive amino group" refers to an amino group that is capable of chemically reacting with another chemical functionality, such as a hydroxy group of a monophosphate moiety.

The term "linkages" refers to chemical bonds, which can be ionic and/or covalent in nature, between the entities named.

The phrase "terminal monophosphate functionality per absorbable polyester chain" indicates that each end of the polyester chain can have a monophosphate functionality.

Members of the monophosphate family of polyesters which are miscible in cyanoacrylate esters can be used as an acidic excipient in absorbable tissue adhesive compositions comprising alkoxyalkyl cyanoacrylates. The acidic functionality of these polyesters can stabilize the cyanoacrylate in the presence of trace amounts of environmental contaminants that are basic in nature.

The term "low melting polyester" means that the polyester has a melting range of between about 25 °C to 120 °C.

The term "crystallinity" refers to the fraction of the solid that exhibits 3-dimensional order that is maintained until the material is heated to a temperature at or above the melting temperature where the material converts to a liquid.

### Detailed Description

The present invention is directed to monophosphate analogues of several types of hydroxylic oligomers and polymers, which can be liquids, amorphous solids, or crystalline materials at room temperature. The free -P(O)(OH)₂ functionality of the phosphorylated derivatives of the oligomeric and polymeric systems is capable of conjugating, ionically and/or covalently, with basic amine groups of bioactive agents including peptides and proteins. The phosphorylation can be conducted on molten polyester or solid microparticulate polyesters. The phosphorylated substrates of this invention are used to form novel ionic conjugates with amino-acids, polypeptides, and proteins or any organic compound that has at least one available interactive amine group.

The hydroxylic oligomers and polymers that are the subject of this invention include hydroxy terminated polyesters (HTPE). The polyesters (HTPE) can be prepared by the ring opening polymerization of lactones (such as glycolide, lactide, ε-caprolactone, p-dioxanone) and/or cyclic carbonate (such as trimethylene carbonate) in the presence of an inorganic or organometallic catalyst (such as stannous octoate) and a hydroxyl-bearing initiator (such as 1,6-hexanediol, 1,3-propanediol and diethylene glycol) under conditions that are commonly used in the art for ring opening polymerization (see Shalaby, S.W., et al., "Absorbable Polyesters" in Biomedical Polymers Designed to Degrade, Chapter 1, Shalaby, S.W. Ed. Hansen Publ., NY 1994). The resulting polyesters, copolyesters, or copolyester-carbonates can be in the form of a liquid, amorphous or highly crystalline materials having at least one hydroxyl group per polymeric chain. Highly crystalline solids of polyesters of this invention can be reduced in size to form microparticles having an average diameter of 0.1 to 100 microns.

Conversion of the hydroxyl-bearing oligomers and polymers of the types described herein is achieved by phosphorylating the hydroxyl groups thereof, using reactive phosphoric acid derivatives, such as pyrophosphoric acid. Stoichiometric or excess amounts of the phosphorylating agent is used. The reaction is carried out with or without an unreactive solvent and at a temperature ranging from about 10° C to 150° C for a period of several minutes to several hours, depending on the chemical structure of the reacting substrate. At the conclusion of the reaction, the product is fractionated to remove any excess reagent and by-products. Upon using pyrophosphoric acid, the resulting reaction mixture is dissolved in a water-miscible solvent, such as acetone. The acetone solution is then added to stirring ice-water. This is followed by isolating the solid phosphorylated product by centrifugation or filtration. After rinsing the product sufficiently, it is dried under reduced pressure. When crystalline microparticulate hydroxylic substrates are used, the reaction conditions can be adjusted to insure that the microparticles maintain their original dimensions after being phosphorylated. The reaction of the microparticulate can be conducted with or without the use of a liquid non-reactive organic medium. Pyrophosphoric acid is a typical example of the reactive acid derivatives that can be used to insert -O-P(O)(OH)₂ functionality into the hydroxyl-bearing substrate. Examples of other phosphorylating agents that can be used in the present invention include methyl monophosphate, ethyl monophosphate and phenyl monophosphate. To form conjugates of the different polymer monophosphates, the individual polymer monophosphate is dissolved or suspended in a suitable medium "A", such as water or a mixture of water and acetonitrile, and allowed to interact with a basic bioactive agent present in an aqueous solution that is miscible in "A". Depending on the chemistry of the bioactive agent, a basic inorganic reagent may be required to present the bioactive agent in its free basic form. In case of a peptide salt, such as a peptide acetate, an inorganic base, such as sodium bicarbonate, can be used to abstract the acetate ions from the peptide and allow its free base to conjugate, ionically and/or covalently, with the monophosphate group of the oligomeric or polymeric substrates.

A microparticle of the present invention is made by micronizing a polymer by initially grinding it using a Knife-grinder. The polymer is then micronized in an Aljet Micronizer using a pressurized dry nitrogen stream. The mean particle diameter size is analyzed in a Malvern Mastersizer/E using a volume distribution model and 200/5 cS silicone oil as dispersant.

The conjugate microparticles of this invention can be administered to a patient via administration routes well known to those of ordinary skill in the art, such as parenteral administration, oral administration or topical administration. Preferably, it is administered as a powder or a suspension via intranasal route or as an inhalant through the pulmonary system. When it is administered parenterally it is preferable that it is administered as a dispersion in an isotonic aqueous medium or in a non-aqueous, absorbable gel-forming liquid polyester as described in U.S. Patent No. 5,612,052, the contents of which are incorporated herein by reference. The formulations comprising conjugate microparticles of the present invention can also include a variety of optional components. Such components include, but are not limited to, surfactants, viscosity controlling agents, medicinal agents, cell growth modulators, dyes, complexing agents, antioxidants, other polymers such as carboxymethyl cellulose, gums such as guar gum, waxes/oils such as castor oil, glycerol, dibutyl phthalate and di(2-ethylhexyl)phthalate and the like. If used, such optional components comprise from about 0.1% to about 20%, preferably from about 0.5% to about 5% of the total formulation.

The effective dosages of a conjugate microparticles of the present invention to be administered to a patient can be determined by the attending physician or veterinarian and will be dependent upon the proper dosages contemplated for the peptide and/or bioactive agent conjugated in the microparticles. Such dosages will either be known or can be determined by one of ordinary skill in the art.

The disclosure of each of the references cited herein are incorporated herein by reference.

The following examples are provided for illustrative purposes and the teachings therein are not meant to limit the scope of the present invention.

### Example 1

### Preparation of a Hydroxy-Terminated Polyester:

CEG-1 was made from caprolactone (137.3 g, 1.204 mole) and diethylene glycol (12.7 g, 0.12 mole) using stannous octoate (Sigma Chemical Co., St. Louis, MO), as a catalyst, at 5000:1 mole ratio of monomer/catalyst (1.3 ml of 0.2M toluene solution). The flask was charged and set up with a mechanical stirrer. The reactants were heated for about 12 hours at about 150° C under dry argon. The polymer was isolated and purified by precipitating an acetone solution of the reaction mixture in ice water. The resulting polymer was isolated, dried, and then analyzed for identity composition, molecular weight, and thermal properties using IR, NMR, GPC, and DSC, respectively. Unreacted monomer was distilled under reduced pressure at about 120°C.

Substituting 1,3-propanediol for diethylene glycol at different monomer/initiator ratios, a number of hydroxy terminated polymers, having a range of molecular weights, were prepared according to the procedure described for Example 1. These include CPD-1 and CPD-2, which are based on ε-caprolactone and 1,3-propanediol.

Using a mixture of 70/30 I-lactide/glycolide, 85/15 dl-lactide/glycolide, 70/30 I-lactide/glycolide or 80/20 I-lactide/di-lactide, the respective copolymers, denoted PDLG-1, PDLG-2, PD-100, and PD-101, respectively, were produced substantially according to the procedure described for Example 1 under similar reaction conditions and variable amounts of 1,3-propanediol to achieve the desired molecular weights (Mn) ranging from 2 to 20 kDa.

### Example 2

### Preparation of Phosphorylated Polyesters:

CEG-1-Phos-5 was made from CEG-1 (from Example 1) and pyrophosphoric acid without the aid of a solvent using a 4:1 mole ratio of pyrophosphoric acid (1.3 g) to CEG-1 (5.0 g). The foregoing reaction mixture was allowed to react for about 80 minutes at room temperature. The resulting product was dissolved in 30 ml acetone and precipitated in ice water. The polymer was isolated and dried under reduced pressure and then analyzed as described in Example 1. In addition, the phosphorous content was determined by elemental analysis. The equivalent weight of the phosphorylated product was determined by acidimetry.

Following the foregoing procedure for making CEG-1-Phos-5, two other batches of phosphorylated CEG-1 were prepared, namely CEG-1-Phos-6 and CEG-1-Phos-7. Similarly, CPD-2 was phosphorylated to produce CPD-2-Phos-1 and copolymers, PLDG-1, PDLG-2, PD-100, and PD-101 to produce PLDG-1-Phos-1, PDLG-1-Phos-1, PD-100-Phos-1 and PD-101-Phos-1, respectively, all substantially according to the procedure for making CEG-1-Phos-5.

### Example 3

### Preparation of Phosphorylated Polyester/Polypeptide Conjugates:

CON-P3 was made from Peptide A and CEG-1-Phos-5A (another batch of CEG-1-Phos-5). Thus, CEG-1-Phos-5A (0.9974g) was dissolved in 10 ml of acetonitrile. This solution was filtered through a syringe filter in order to remove traces of insoluble polymer particles. Peptide A (199 mg) was dissolved in 2 ml of water. Based on the acetate content of the Peptide A, 25 mg of sodium carbonate was added to the polymer solution in acetonitrile to exchange with the acetate in the peptide. Peptide A solution was then added to the polymer solution dropwise. After the entire peptide solution was added over about a ten minute interval, the resulting solution was allowed to stir for about 0.5 hour. The solution was then precipitated into ice cold salt water and centrifuged to collect the product. The latter was rinsed with distilled water and recentrifuged. The product was dried under vacuum. The resulting conjugate was analyzed for its peptide content using elemental analysis for nitrogen.

Following a similar procedure as described for Example 3, the conjugates described in Table I were prepared from the designated individual peptides and phosphorylated polyesters at the noted ratios and precipitation media (PM, water or isopropyl alcohol).

### Example 4

### Preparation of Crystalline Hydroxy-Terminated Polyglycolide Microparticles:

PDGLY-1 was made as a solid crystalline material using about 25:1 mole ratio of glycolide to 1,3-propanediol. Glycolide (200 g, 1.724 mole) was melted in a flame-dried flask under argon and 1,3-propanediol (5.249 g, 69.1 mmole) was added. Stannous octoate (0.575 ml of 0.2M in toluene) was placed in the flask containing the molten reactants and the temperature was raised to about 160 °C. After approximately 30 minutes, the polymerization was concluded and the temperature was lowered to about 110 °C. Unreacted monomer was removed by distillation at about 120 °C under reduced pressure. The product was then ground and micronized as described hereinabove.

In a similar manner as the procedure for Example 4, PD-102 was made from a 15/1 mixture of glycolide and 2,3-propanediol.

### Example 5

### Preparation of Surface Phosphorylated Polyglycolide Microparticles:

PDGLY-1-Phos-2 was made by melting 9.7 g of pyrophosphoric acid at about 60° C and adding, while stirring under dry argon, 4.4 g of PDGLY-1. The reaction was continued for about three hours at the same temperature. The product was cooled to about 10° C and then mixed with 20 ml of cold water. The mixture was sonicated for about 5 minutes, washed three times with water, and then dried under reduced pressure. The product was analyzed for its phosphorous content and Tₘ using elemental analysis and DSC, respectively.

In an analogous manner, PD-102 was converted to PD102-Phos1 substantially according to the procedure for making PDGLY-1-Phos-2.

### Example 6

### Preparation of Typical Polypeptide Conjugates Using Phosphorylated Polyglycolide Microparticles:

PICP3 was made from PDGLY1-Phos2 and Peptide B (50 mg) dissolved in 2 ml of a 50:50 acetonitrile/water mixture. A 20 µl aliquot of the peptide solution was removed as a control sample. PDGLY1-Phos2 (502 mg) was added to the vial containing the rest of the peptide solution and sonicated for about five minutes and then stirred for about two hours. The product was isolated by centrifugation and then dried under reduced pressure. The supernatant liquid was analyzed by HPLC to determine the amount of peptide bound to the powder. HPLC results indicated that 6.7%, by weight, of peptide was bound. The peptide content was found to be 7.2% by elemental analysis for nitrogen.

In an analogous manner as the foregoing procedure, PD102-Phos1 was conjugated with Peptide B to produce PICP-4.

### Example 7

### Preparation of Endo-Chain Carboxylated 85/15 Poly(dl-lactide-co-glycolide) (TR-100):

An 85/15 (molar) mixture of dl-lactide and glycolide was polymerized following essentially the same procedure described in Example 1 but using L-tartaric acid as the initiator (at a molar ratio of 50/1 lactone/tartaric acid) and stannous octoate as a catalyst (at a monomer to catalyst ratio of 5000/1).

### Example 8

### Phosphorylation of TR-100 to produce TR101-Phos2:

The phosphorylation was conducted as described in Example 2, using a mixture of TR-100 (15 g) and pyrophosphoric acid (1.244g).

### Example 9

### Ionic Conjugation of TR100-Phos1 to Produce CON-P9, CON-P12, and CON-P15:

Using a similar procedure to that described for Example 3, Peptide B (373 mg) was reacted with 1.5 g TR100-Phos1 to produce CON-P9. Similarly, CON-P12 was prepared using Peptide A (400 mg) and TR10o-Phos1 (2.5 g). In preparing CON-P15, cold 2-propanol was used as the precipitating medium producing a conjugate based on Peptide B (400 mg) and TR100-Phos1 (2.5g).

### Example 12

### Preparation of a Tissue Adhesive Composition:

The phosphorylated polyester from Example 2 (0.5 g) was dissolved in methoxypropyl cyanoacrylate (9.5 g) and the resulting liquid composition was stored at room temperature in an untreated (not washed with acid) glass vial for several days and showed no signs of polymerization (as indicated by no visible change in viscosity). Applying such tissue adhesive composition to a moist goat skin led to the formation of a compliant (flexible) tissue adhering film in about one minute.

**Table I. Experimental Data for the Preparation of Typical Conjugates**

| Conjugate | Phosphorylated Polyester | Peptide | Phosphorylated Polyester/Peptide | PM* |
|---|---|---|---|---|
| CON-P1 | CEG1-Phos5 | B | 1.5g/373mg | H₂O |
| CON-P7 | CEG1-Phos6 | B | 2.0g/300mg | H₂O |
| CON-P10 | CEG1-Phos7 | A | 3.0g/758mg | H₂O |
| CON-P11 | CEG1-Phos7 | A | 1.5g/190mg | IPA |
| CON-P13 | CPD2-Phos1 | A | 3.0g/700mg | H₂O |
| CON-P14 | CPD2-Phos1 | A | 3.0g/500mg | IPA |
| CON-P4 | PDLG2-Phos1B | B | 1.5g/373mg | H₂O |
| CON-P5 | PDLG2-Phos1B | C | 2.0g/333mg | H₂O |
| CON-P6 | PDLG2-Phos1B | A | 1.5g/300mg | H₂O |
| CON-P15 | TR100-Phos1 | A | 2.5g/400mg | IPA |
| CON-P8 | PD100-Phos1 | B | 1.5g/373mg | H₂O |
| CON-P16 | PD101-Phos1 | B | 1.5g/373mg | H₂O |
| PIC-P3 | PDGLY1-Phos2 | B | 0.5g/50mg | N/A |
| PIC-P4 | PD102-Phos1 | B | 0.5g/50mg | N/A |

| | | | | |
|---|---|---|---|---|
| *PM= Precipitation medium | | | | |

**Table II. Composition and Properties of Conjugates and Their Precursors**

| Analytical Data | | | | | | | |
|---|---|---|---|---|---|---|---|
| Polyester Number | Yield % | GPC | | DSC Tm | Eq. Wt.* | Phos. % | Peptide %** |
| | | Mn | Mw | | | | |
| TR-100 | 81.2 | 8582 | 22,384 | | 1333 | | |
| PD-100 | 86.5 | 5205 | 16.3x10³ | -- | -- | -- | -- |
| PD-101 | 88.3 | 13.3x10³ | 18.6x10³ | -- | -- | -- | -- |
| PD-102 | 99.1 | -- | -- | -- | -- | -- | -- |
| CEG-1 | 96.0 | 2732 | 3134 | 47.5 | 7395 | | |
| CPD-1 | 91.9 | 2732 | 3595 | N/A | 22,440 | | |
| CPD-2 | 96.6 | 5788 | 8527 | 58.3 | 39,324 | | |
| PDLG-2 | 93.8 | 10x10³ | 17x10³ | -- | -- | -- | -- |
| PDLG4-1 | 95.0 | 9388 | 13614 | | | | |
| PDGLY-1 | 77.7 | -- | -- | -- | -- | -- | -- |

| Phosphorylated Number/Precursor Used | | | | | | | |
|---|---|---|---|---|---|---|---|
| CPD2-Phos1/CPD-2 | 19.2 | 5376 | 24x10³ | 53.6 | 745 | 0.85 | -- |
| TR100-Phos1/TR-100 | 81.8 | 9067 | 29,656 | -- | 988 | 0.68 | |
| PD100-Phos1/PD-100 | 89.0 | 6108 | 8776 | 39.3 | 1344 | 0.56 | |
| PDLG2-Phos1 B/PDLG-2 | 97.2 | 8833 | 14 x10³ | 46.2 | 2624 | 0.07 | -- |
| PD101-Phos1/PD-101 | 70.0 | 8535 | 16,188 | -- | 1110 | 0.83 | |
| PD102-Phos1/PD-102 | 76.8 | -- | -- | -- | -- | 0.98 | -- |
| CEG1-PhosS/CEG-1 | 60.3 | 2926 | 3734 | 53.5 | 1339 | 1.11 | |
| CEG1-Phos6/CEG-1 | 69.6 | 4217 | 8825 | 41.4 | 960 | 2.11 | |
| CEG1-Phos7/CEG-1 | 52.0 | 4345 | 8593 | -- | 1020 | 1.85 | |
| PDLG1-Phos1/PDLG-1 | 78.1 | 8703 | 12,767 | -- | 2810 | 0.19 | |
| PDGLY1-Phos2/PDGLY-1 | 88.6 | -- | -- | -- | -- | 0.60 | |

| Peptide Number/ Precursor Used | | | | | | | |
|---|---|---|---|---|---|---|---|
| CON-P9/TR100-Phos1 | 43.0 | | | -- | | | B (10.96) |
| CON-P12/TR100-Phos1 | 11.8 | | | -- | | | A (21.4) |
| CON-P8/PD100-Phos1 | 73 | | | 54.4 | | | B (11.25) |
| CON-P4/PDLG2-Phos1B | 63.2 | | | 53.8 | | | B (4.9) |
| CON-P5/PDLG2-Phos1B | 73.5 | | | 48.0 | | | C (4.7) |
| CON-P6/PDLG2-Phos1B | 78.6 | | | 54.9 | | | A (4.8) |
| CON-P1/CEG1-Phos5 | 63.4 | | | | | | B (18.5) |
| CON-P3/CEG1-Phos5 | 82.3 | | | | | | A (12.8) |
| CON-P7/CEG1-Phos6 | 33 | | | 47.8 | | | B (12.66) |
| CON-P2/PDLG1-Phos1 | 62.4 | | | | | | B (5.4) |
| CON-P11/CEG1-Phos7 | 70.7 | | | | | | A (9.94) |
| CON-P10/CEG1-Phos7 | 6.8 | | | | | | A (5.69) |
| CON-P15/TR100-Phos1 | 72.4 | | -- | | | | A (6.2) |
| CON-P13/CPD2-Phos1 | 5.1 | -- | -- | | -- | -- | A (8.78) |
| CON-P14/CPD2-Phos1 | 75.1 | -- | -- | 57.0 | -- | -- | A (9.5) |
| CON-P8/PD100-Phos1 | 73 | -- | -- | 54.4 | -- | -- | B (11.25) |
| CON-P4/PLG2-Phos1B | 63.2 | | -- | 53.8 | -- | -- | B (4.9) |
| CON-P5/PLG2-Phos1B | 73.5 | -- | -- | 48.0 | -- | -- | C (4.7) |
| CON-P6/PLG2-Phos1B | 78.6 | -- | -- | 54.9 | -- | -- | A (4.8) |
| CON-P16/PD101-Phos1 | 61.6 | -- | -- | -- | -- | -- | B (4.8) |
| PIC-P3/PDGLY1-Phos2 | 74 | -- | -- | -- | -- | -- | B (7.2) |
| PIC-P4/PD102-Phos1 | 87.2 | -- | -- | -- | -- | -- | B (5.6) |
| CONG-P100/GCD-Phos3 | 70.3 | -- | -- | -- | -- | -- | B (4.3) |

| | | | | | | | |
|---|---|---|---|---|---|---|---|
| * Equivalent weight was determined by titration for acid groups ** Based on elemental analysis for nitrogen. | | | | | | | |

**Table III. Release Data**

| **Conjugate** | **Analyte** | ***In Vitro* Study, Cumulative % Released at Specific Intervals** | | | | | | | ***In Vivo* Release* (ng/ml)/on day** | | | | | |
|---|---|---|---|---|---|---|---|---|---|---|---|---|---|---|
| | Peptide (%) | Day 5 | Day 10 | Day 15 | Day 20 | Day 25 | Day 30 | Day 40 | 0.25 | 5 | 10 | 15 | 23 | 30 |
| CON-P1 | B (18.5)** | 2.3 | 4.4 | 6.3 | 9.45 | 11.5 | 12.8 | 13.9 | 11.5 | 6.4 | 0.8 | 0.2 | -- | -- |
| CON-P2 | B (5.4) | 11.9 | 18.7 | 27.4 | 40.1 | 46.8 | 51.0 | 55.3 | -- | -- | -- | -- | -- | -- |
| CON-P3 | A (12.8) | 20.2 | 29.4 | 30.3 | 39.2 | 43.7 | 46.5 | 46.9 | -- | -- | -- | -- | -- | -- |
| CON-P4 | B (4.9) | 11.7 | 18.5 | 25.4 | 30.9 | 36.1 | 39.1 | 42.8 | 12.8 | 1.9 | < 0.1 | -- | -- | -- |
| CON-P5 | C (4.7) | 1.6 | 2.3 | 2.7 | 3.0 | 3.4 | 3.8 | 4.0 | 2.04 | 0.18 | 0.23 | 0.56 | 0.15 | |
| CON-P6 | A (4.8) | 2.8 | 3.4 | 3.4 | 4.1 | 4.6 | -- | -- | -- | -- | -- | -- | -- | -- |
| CON-P7 | B (12.66) | 3.2 | 4.9 | 6.2 | -- | -- | -- | -- | -- | -- | -- | -- | -- | -- |
| CON-P8 | B (11.25) | 14.9 | 19.4 | 24.7 | -- | -- | -- | -- | 15.4 | 8.0 | 4.6 | 1.0 | 0.2 | -- |
| CON-P9 | B (10.96) | 10.8 | 13.5 | 19.3 | -- | -- | -- | -- | 9.6 | 3.7 | 3.0 | < 0.1 | -- | -- |
| CON-P10 | A (5.7) | | | | | | | | -- | -- | -- | -- | -- | -- |
| CON-P11 | A (9.9) | | | | | | | | -- | -- | -- | -- | -- | -- |
| CON-P12 | A (21.4) | 37.0 | 55.4 | 60.9 | 62.4 | | | | -- | -- | -- | -- | -- | -- |
| CON-P13 | A (8.8) | | | | | | | | -- | -- | -- | -- | -- | -- |
| CON-P14 | A (9.5) | 27.8 | 38.0 | | | | | | -- | -- | -- | -- | -- | -- |
| CON-P15 | A (5.25) | 16.6 | 19.9 | -- | -- | -- | -- | -- | -- | -- | -- | -- | -- | -- |
| CON-P16 | B (4.8) | -- | -- | -- | -- | -- | -- | -- | -- | -- | -- | -- | -- | -- |
| PIC-P3 | B (6.7) | 2.1 | 2.9 | 3.2 | 3.6 | 3.8 | 4.1 | 5.8 | -- | -- | -- | -- | -- | -- |
| PIC-P4 | B (6.12) | | | | | | | | -- | -- | -- | -- | -- | -- |
| CONG-P100 | B (4.3) | 5.7 | 12.1 | 12.8 | -- | -- | | | -- | -- | -- | -- | -- | -- |

| | | | | | | | | | | | | | | |
|---|---|---|---|---|---|---|---|---|---|---|---|---|---|---|
| * The release study was conducted in rats and the release profile was monitored by determining the peptide concentration in serum at different time periods using radio immunoassay. ** Only half the usual dose was administered to rats. | | | | | | | | | | | | | | |

## Claims

1. An absorbable polyester with at least one monophosphate functionality per absorbable polyester chain, wherein the polymer chain is covalently linked to phosphoric acid by a single phosphate bond leaving two additional acidic groups available for conjugation or neutralization and wherein the polyester chain comprises one or more monomers selected from the group consisting of L-lactic acid, D-lactic acid, DL-lactic acid, malic acid, citric acid, tartaric acid, alkylene oxalate, cycloalkylene oxalate, alkylene succinate, β-hydroxybutyrate, glycolide, glycolic acid, L-lactide, D-lactide, DL-lactide, meso-lactide, trimethylene carbonate, p-dioxanone, 1,5-dioxepan-2-one and 1,4-dioxepan-2-one and any optically active isomers, racemates, or copolymers thereof.

2. A conjugate comprising an absorbable polyester with at least one monophosphate functionality per absorbable polyester chain, wherein the polymer chain is covalently linked to phosphoric acid by a single phosphate bond leaving two additional acidic groups available for conjugation or neutralization and wherein the polyester chain comprises one or more monomers selected from the group consisting of L-lactic acid, D-lactic acid, DL-lactic acid, malic acid, citric acid, tartaric acid, ε-hydroxy caproic acid, ε-caprolactone, alkylene oxalate, cycloalkylene oxalate, alkylene succinate, β-hydroxybutyrate, glycolide, glycolic acid, L-lactide, D-lactide, DL-lactide, meso-lactide, trimethylene carbonate, p-dioxanone, 1,5-dioxepan-2-one and -1,4-dioxepan-2-one and any optically active isomers, racemates, or copolymers thereof and a peptide and/or a bioactive agent, where the peptide and bioactive agent have at least one interactive amino group, wherein the monophosphate functionality forms a linkage with the amino group.

3. A conjugate according to claim 2 wherein the peptide is selected from the group consisting of p-Glu-His-Trp-Ser-Tyr-D-Trp-Leu-Arg-Pro-Gly-NH₂, H-β-D-Nal-Cys- Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-Cys-Thr-NH₂ where the two Cys are bonded by a disulfide bond, or a pharmaceutically acceptable salt thereof.

4. A solid absorbable microparticle which comprises the absorbable polyester according to claim 1 and having a surface, wherein more than one percent of the monophosphate functionality resides on the surface of the absorbable microparticle.

5. A conjugate comprising the absorbable microparticle according to claim 4 and a peptide and/or a bioactive agent, where the peptide and bioactive agent have at least one interactive amino group, wherein the monophosphate functionality on the surface of the absorbable microparticle forms a linkage with the amino group.

6. A conjugate comprising a solid absorbable microparticle which comprises an absorbable polyester with at least one monophosphate functionality per absorbable polyester chain, wherein the polymer chain is covalently linked to phosphoric acid by a single phosphate bond leaving two additional acidic groups available for conjugation or neutralization and wherein the polyester chain comprises one or more monomers selected from the group consisting of L-lactic acid, D-lactic acid, DL-lactic acid, malic acid, citric acid, tartaric acid, ε-hydroxy caproic acid, ε-caprolactone, alkylene oxalate, cycloalkylene oxalate, alkylene succinate, β-hydroxybutyrate, glycolide, glycolic acid, L-lactide, D-lactide, DL-lactide, meso-lactide, trimethylene carbonate, p-dioxanone, 1,5-dioxepan-2-one and 1,4-dioxepan-2-one and any optically active isomers, racemates, or copolymers thereof and having a surface, wherein more than one percent of the monophosphate functionality resides on the surface of the absorbable microparticle and a peptide and/or a bioactive agent, where the peptide and bioactive agent have at least one interactive amino group, wherein the monophosphate functionality on the surface of the absorbable microparticle forms a linkage with the amino group.

7. A conjugate according to claim 5 or 6 wherein the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-Cys-Thr-NH₂ where the two Cys are bonded by a disulfide bond, or a pharmaceutically acceptable salt thereof.

8. An absorbable polyester according to claim 1 further comprising one or more polyethylene glycol segments covalently linked to said polyester.

9. A conjugate comprising an absorbable polyester according to claim 8 and a peptide and/or a bioactive agent, where the peptide and bioactive agent have at least one interactive amino group, wherein the monophosphate functionality forms a linkage with the amino group.

10. A conjugate according to claim 9 wherein the peptide is selected from the group consisting of p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ where the two Cys are bonded by a disulfide bond, N-hydroxyethylpiperazinyl-acetyl-D-Phe-Cys-Tyr-D-Trp-Lys-Abu-Cys-Thr-NH₂ where the two Cys are bonded by a disulfide bond and N-hydroxyethylpiperazinyl-ethylsulfonyl-Phe-Cys-Tyr-D-Trp-Lys-Abu-Cys-Thr-NH₂ where the two Cys are bonded by a disulfide bond, or a pharmaceutically acceptable salt thereof.

11. A pharmaceutical composition comprising a conjugate according to any one of claims 2, 3, 5, 6, 7, 9 or 10 and a pharmaceutically acceptable carrier.

## Patentansprüche

1. Absorbierbarer Polyester mit mindestens einer Monophosphatfunktionalität pro absorbierbarer Polyesterkette, wobei die Polymerkette mittels einer einzigen Phosphatbindung kovalent an Phosphorsäure gebunden ist, wodurch zwei zusätzliche Säuregruppen für Konjugation oder Neutralisation zur Verfügung stehen, und wobei die Polyesterkette ein oder mehrere Monomere ausgewählt aus der Gruppe bestehend aus L-Milchsäure, D-Milchsäure, DL-Milchsäure, Äpfelsäure, Zitronensäure, Weinsäure, Alkylenoxalat, Cycloalkylenoxalat, Alkylensuccinat, β-Hydroxybutyrat, Glykolid, Glykolsäure, L-Lactid, D-Lactid, DL-Lactid, meso-Lactid, Trimethylencarbonat, p-Dioxanon, 1, 5-Dioxepan-2-on und 1, 4-Dioxepan-2-on und irgendwelchen optisch aktiven Isomeren, Racematen oder Copolymeren derselben umfasst.

2. Konjugat, das einen absorbierbaren Polyester mit mindestens einer Monophosphatfunktionalität pro absorbierbarer Polyesterkette umfasst, wobei die Polymerkette mittels einer einzigen Phosphatbindung kovalent an Phosphorsäure gebunden ist, wodurch zwei zusät-zliche Säuregruppen für Konjugation oder Neutralisation zur Verfügung stehen, und wobei die Polyesterketten ein oder mehrere Monomere ausgewählt aus der Gruppe bestehend aus L-Milchsäure, D-Milchsäure, DL-Milchsäure, Äpfelsäure, Zitronensäure, Weinsäure, ε-Hydroxycapronsäure, ε-Caprolacton, Alkylenoxalat, Cycloalkylenoxalat, Alkylensuccinat, β-Hydroxybutyrat, Glykolid, Glykolsäure, L-Lactid, D-Lactid, DL-Lactid, meso-Lactid, Trimethylencarbonat, p-Dioxanon, 1,5-Dioxepan-2-on und 1,4-Dioxepan-2-on und irgendwelchen optisch aktiven Isomeren, Racematen oder Copolymeren derselben und ein Peptid und/oder bioaktives Mittel umfasst, wobei das Peptid und bioaktive Mittel mindestens eine interaktive Aminogruppe aufweisen, wobei die Monophosphatfunktionalität eine Bindung mit der Aminogruppe bildet.

3. Konjugat nach Anspruch 2, bei dem das Peptid ausgewählt ist aus der Gruppe bestehend aus p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂, wobei die beiden Cys durch eine Disulfidbindung gebunden sind, N-Hydroxyethylpiperazinyl-acetyl-D-Phe-Cys-Tyr-D-Trp-Lys-Abu-Cys-Thr-NH₂, wobei die beiden Cys durch eine Disulfidbindung gebunden sind, und N-Hydroxyethylpiperazinyl-ethylsulfonyl-Phe-Cys-Tyr-D-Trp-Lys-Abu-Cys-Thr-NH₂, wobei die beiden Cys durch eine Disulfidbindung gebunden sind, oder einem pharmazeutisch annehmbaren Salz derselben.

4. Festes absorbierbares Mikroteilchen, das den absorbierbaren Polyester gemäß Anspruch 1 umfasst und eine Oberfläche aufweist, bei der mehr als 1 % der Monophosphatfunktionalität an der Oberfläche des absorbierbaren Mikroteilchens vorliegen.

5. Konjugat, das das absorbierbare Mikroteilchen gemäß Anspruch 4 und ein Peptid und/oder ein bioaktives Mittel umfasst, wobei das Peptid und bioaktive Mittel mindestens eine interaktive Aminogruppe aufweisen, wobei die Monophosphatfunktionalität an der Oberfläche des absorbierbaren Mikroteilchens eine Bindung mit der Aminogruppe bildet.

6. Konjugat, das ein absorbierbares Mikroteilchen umfasst, das einen absorbierbaren Polyester mit mindestens einer Monophosphatfunktionalität pro absorbierbarer Polyesterkette umfasst, wobei die Polymerkette mittels einer einzigen Phosphatbindung kovalent an Phosphorsäure gebunden ist, wodurch zwei zusätzliche Säuregruppen für Konjugation oder Neutralisation zur Verfügung stehen, und wobei die Polyesterkette ein oder mehrere Monomere ausgewählt aus der Gruppe aus L-Milchsäure, D-Milchsäure, DL-Milchsäure, Äpfelsäure, Zitronensäure, Weinsäure, ε-Hydroxycapronsäure, ε-Caprolacton, Alkylenoxalat, Cycloalkylenoxalat, Alkylensuccinat, β-Hy-droxybutyrat, Glykolid, Glykolsäure, L-Lactid, D-Lactid, DL-Lactid, meso-Lactid, Trimethylencarbonat, p-Dioxanon, 1,5-Dioxepan-2-on und 1,4-Dioxepan-2-on und irgendwelchen optisch aktiven Isomeren, Racematen oder Copolymeren derselben umfasst, und eine Oberfläche aufweist, bei der mehr als 1 % der Monophosphatfunktionalität an der Oberfläche des absorbierbaren Teilchens vorliegt, und ein Peptid und/oder bioaktives Mittel umfasst, wobei das Peptid und bioaktive Mittel mindestens eine interaktive Aminogruppe aufweisen, wobei die Monophosphatfunktionalität an der Oberfläche des absorbierbaren Mikroteilchens eine Bindung mit der Aminogruppe bildet.

7. Konjugat nach Anspruch 5 oder 6, bei dem das Peptid ausgewählt ist aus der Gruppe bestehend aus p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂, wobei die beiden Cys durch eine Disulfidbindung gebunden sind, N-Hydroxyethylpiperazinyl-acetyl-D-Phe-Cys-Tyr-D-Trp-Lys-Abu-Cys-Thr-NH₂, wobei die beiden Cys durch eine Disulfidbindung gebunden sind, und N-Hydroxyethylpipe-razinyl-ethylsulfonyl-Phe-Cys-Tyr-D-Trp-Lys-Abu-Cys-Thr-NH₂, wobei die beiden Cys durch eine Disulfidbindung gebunden sind, oder einem pharmazeutisch annehmbaren Salz derselben.

8. Absorbierbarer Polyester nach Anspruch 1, der ferner ein oder mehrere Polyethylenglykolsegmente umfasst, die kovalent an den Polyester gebunden sind.

9. Konjugat, das einen absorbierbaren Polyester gemäß Anspruch 8 und ein Peptid und/oder bioaktives Mittel umfasst, wobei das Peptid und bioaktive Mittel mindestens eine interaktive Aminogruppe aufweisen, wobei die Monophosphatfunktionalität eine Bindung mit der Aminogruppe bildet.

10. Konjugat nach Anspruch 9, bei dem das Peptid ausgewählt ist aus der Gruppe bestehend aus p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂, wobei die beiden Cys durch eine Disulfidbindung gebunden sind, N-Hydroxyethylpiperazinyl-acetyl-D-Phe-Cys-Tyr-D-Trp-Lys-Abu-Cys-Thr-NH₂, wobei die beiden Cys durch eine Disulfidbindung gebunden sind, und N-Hydroxyethylpiperazinyl-ethylsulfonyl-Phe-Cys-Tyr-D-Trp-Lys-Abu-Cys-Thr-NH₂, wobei die beiden Cys durch eine Disulfidbindung gebunden sind, oder einem pharmazeutisch annehmbaren Salz derselben.

11. Pharmazeutische Zusammensetzung, die ein Konjugat gemäß einem der Ansprüche 2, 3, 5, 6, 7, 9 oder 10 und einen pharmazeutisch annehmbaren Träger umfasst.

## Revendications

1. Polyester absorbable ayant au moins une fonctionnalité monophosphate par chaîne polyester absorbable, dans lequel la chaîne polymère est liée de façon covalente à un acide phosphorique par une simple liaison phosphate laissant deux groupes acides supplémentaires disponibles pour une conjugaison ou une neutralisation et dans lequel la chaîne de polyester comprend un ou plusieurs monomères choisis dans le groupe constitué par l'acide L-lactique, l'acide D-lactique, l'acide DL-lactique, l'acide malique, l'acide citrique, l'acide tartrique, un oxalate d'alkylène, un oxalate de cycloalkylène, un succinate d'alkylène, le β-hydroxybutyrate, le glycolide, l'acide glycolique, le L-lactide, le D-lactide, le DL-lactide, le méso-lactide, le carbonate de triméthylène, la p-dioxanone, la 1,5-dioxépan-2-one et la 1,4-dioxépan-2-one et tous isomères optiquement actifs, racémates ou copolymères de ceux-ci.

2. Conjugué comprenant un polyester absorbable ayant au moins une fonctionnalité monophosphate par chaîne de polyester absorbable, dans lequel la chaîne polymère est liée de façon covalente à un acide phosphorique par une simple liaison phosphate laissant deux groupes acides supplémentaires disponibles pour une conjugaison ou une neutralisation et dans lequel la chaîne de polyester comprend un ou plusieurs monomères choisis dans le groupe constitué par l'acide L-lactique, l'acide D-lactique, l'acide DL-lactique, l'acide malique, l'acide citrique, l'acide tartrique, l'acide e-hydroxy caproïque, la ε-caprolactone, un oxalate d'alkylène, un oxalate de cycloalkylène, un succinate d'alkylène, le β-hydroxybutyrate, le glycolide, l'acide glycolique, le L-lactide, le D-lactide, le DL-lactide, le méso-lactide, le carbonate de triméthylène, la p-dioxanone, la 1,5-dioxépan-2-one et la 1,4-dioxépan-2-one et tous isomères optiquement actifs, racémates ou copolymères de ceux-ci et un peptide et/ou un agent bioactif, le peptide et l'agent bioactif ayant au moins un groupe amino interactif, la fonctionnalité monophosphate formant une liaison avec le groupe amino.

3. Conjugué selon la revendication 2, dans lequel le peptide est choisi dans le groupe constitué par p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ où les deux Cys sont liés par une liaison disulfure, N-hydroxyéthylpipérazinyl-acétyl-D-Phe-Cys-Tyr-D-Trp-Lys-Abu-Cys-Thr-NH₂ où les deux Cys sont liés par une liaison disulfure et N-hydroxyéthylpipérazinyl-éthylsulfonyl-Phe-Cys-Tyr-D-Trp-Lys-Abu-Cys-Thr-NH₂ où les deux Cys sont liés par une liaison disulfure, ou un sel pharmaceutiquement acceptable de ceux-ci.

4. Microparticule absorbable solide qui comprend le polyester absorbable tel que défini à la revendication 1, et ayant une surface, dans laquelle plus d'un pour cent de la fonctionnalité monophosphate réside sur la surface de la microparticule absorbable.

5. Conjugué comprenant la microparticule absorbable, telle que définie à la revendication 4, et un peptide et/ou un agent bioactif, dans lequel le peptide et l'agent bioactif ont au moins un groupe amino interactif, la fonctionnalité monophosphate sur la surface de la microparticule absorbable formant une liaison avec le groupe amino.

6. Conjugué comprenant une microparticule absorbable solide qui comprend un polyester absorbable ayant au moins une fonctionnalité monophosphate par chaîne polyester absorbable, dans lequel la chaîne polymère est liée de façon covalente à un acide phosphorique par une simple liaison phosphate laissant deux groupes acides supplémentaires disponibles pour une conjugaison ou une neutralisation et dans lequel la chaîne polyester comprend un ou plusieurs monomères choisis dans le groupe constitué par l'acide L-lactique, l'acide D-lactique, l'acide DL-lactique, l'acide malique, l'acide citrique, l'acide tartrique, l'acide ε-hydroxy caproïque, la ε-caprolactone, un oxalate d'alkylène, un oxalate de cycloalkylène, un succinate d'alkylène, le β-hydroxybutyrate, le glycolide, l'acide glycolique, le L-lactide, le D-lactide, le DL-lactide, le méso-lactide, le carbonate de triméthylène, la p-dioxanone, la 1,5-dioxépan-2-one et la 1,4-dioxépan-2-one et tous isomères optiquement actifs, racémates ou copolymères de ceux-ci et ayant une surface, dans lequel plus d'un pour cent de la fonctionnalité monophosphate réside sur la surface de la microparticule absorbable et un peptide et/ou un agent bioactif, le peptide et l'agent bioactif ayant au moins un groupe amino interactif, la fonctionnalité monophosphate sur la surface de la microparticule absorbable formant une liaison avec le groupe amino.

7. Conjugué selon l'une des revendications 5 ou 6, dans lequel le peptide est choisi dans le groupe constitué par p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ où les deux Cys sont liés par une liaison disulfure, N-hydroxyéthylpipérazinyl-acétyl-D-Phe-Cys-Tyr-D-Trp-Lys-Abu-Cys-Thr-NH₂ où les deux Cys sont liées par une liaison disulfure et N-hydroxyéthylpipérazinyl-éthylsulfonyl-Phe-Cys-Tyr-D-Trp-Lys-Abu-Cys-Thr-NH₂ où les deux Cys sont liés par une liaison disulfure, ou un sel pharmaceutiquement acceptable de ceux-ci.

8. Polyester absorbable selon la revendication 1, comprenant en outre un ou plusieurs segments de polyéthylène glycol liés de façon covalente audit polyester.

9. Conjugué comprenant un polyester absorbable tel que défini à la revendication 8 et un peptide et/ou un agent bioactif, dans lequel le peptide et l'agent bioactif ont au moins un groupe amino interactif, la fonctionnalité monophosphate formant une liaison avec le groupe amino.

10. Conjugué selon la revendication 9, dans lequel le peptide est choisi dans le groupe constitué par p-Glu-His-Trp-Ser-Tyr-D-Trp-Leu-Arg-Pro-Gly-NH₂, H-β-D-Nal-Cys-Tyr-D-Trp-Lys-Val-Cys-Thr-NH₂ où les deux Cys sont liés par une liaison disulfure, N-hydroxyéthylpipérazinyl-acétyl-D-Phe-Cys-Tyr-D-Trp-Lys-Abu-Cys-Thr-NH₂ où les deux Cys sont liés par une liaison disulfure et N-hydroxyéthylpipérazinyl-éthylsulfonyl-Phe-Cys-Tyr-D-Trp-Lys-Abu-Cys-Thr-NH₂ où les deux Cys sont liées par une liaison disulfure, ou un sel pharmaceutiquement acceptable de ceux-ci.

11. Composition pharmaceutique comprenant un conjugué tel que défini à l'une quelconque des revendications 2, 3, 5, 6, 7, 9 ou 10 et un support pharmaceutiquement acceptable.
